# EUROPEAN PATENT APPLICATION

(11) **EP 4 170 046 A1**
(43) Date of publication of application: **26.04.2023**
(21) Application number: 21830294.1
(22) Date of filing: 23.06.2021
(51) Int. Cl.: C12Q 1/686

(54) **COMPOSITION FOR SEQUENTIAL POLYMERASE CHAIN REACTION, AND GENE AMPLIFICATION METHOD USING SAME**

(30) Priority: 23.06.2020 KR 20200076683
(71) Applicant: Heimbiotek Inc., Seongnam-si, Gyeonggi-do 13486 (KR)
(72) Inventor: LEE, Jae Hoon, Seoul 05698 (KR)
(74) Representative: Privett, Marianne Alice Louise
(86) International application number: PCT/KR2021/007920
(87) International publication number: WO 2021/261928

(57) **Abstract**

The present invention relates to a composition for a sequential polymerase chain reaction and a gene amplification method using the same. The composition for a sequential polymerase chain reaction comprises a template gene, a DNA polymerase, dNTP, a first forward primer, a first reverse primer, a second reverse primer and a third reverse primer so that a sequential polymerase chain reaction can be induced in one container. Unlike conventional nested PCR, a reactant-adding step is omitted in the sequential polymerase chain reaction according to the present invention, and thus the inflow of contaminants from the outside is low, the yield of polymerase chain reaction (PCR) of genes can be increased, and highly-reproducible polymerase chain reaction results can be obtained even if the presence of genes is very low.

## Description

### CROSS-REFERENCE TO RELATED APPLICATION

This application claims priority to and the benefit of Korean Patent Application No. 10-2020-0076683, filed on June 23, 2020, and Korean Patent Application No. 10-2021-0081843, filed on June 23, 2021, the disclosures of which are incorporated herein by reference in their entirety.

### [Technical Field]

The present invention relates to a composition for a sequential polymerase chain reaction and a gene amplification method using the same, and more specifically to a method for continuously performing a polymerase chain reaction by preparing a template gene, DNA polymerase, dNTP, a first forward primer, a first reverse primer, a second reverse primer and a third reverse primer in one container, wherein it is possible to control each polymerase chain reaction according to the temperature, and since it can be performed sequentially in one container, the inflow of contaminants from the outside is low and the yield of the gene polymerase chain reaction (PCR) is increased during gene amplification.

### [Background Art]

Molecular diagnostics is a field for diagnosing diseases by analyzing genes, and it is currently showing the highest growth among *in vitro* diagnosis fields. In fact, as novel viruses, which are presumed to be caused by rapid environmental pollution and climate change, spread greatly, various studies are in progress on molecular diagnostics with the advantages that the highest diagnostic accuracy among *in vitro* diagnostic fields can be secured, and when a new virus emerges, it is possible to quickly check whether a patient is infected.

There are various methods for analyzing base sequence or base variation, but before using all these methods, a large amount of genetic samples must be obtained. Currently, the most commonly used method by researchers to obtain a genetic sample is a polymerase chain reaction (hereinafter, referred to as "PCR") using a DNA polymerase. In this method, only the desired region of a gene can be amplified by designing a primer capable of binding to a template. A primer is designed by determining the region to be amplified from the gene, and determining the nucleotide sequence capable of complementary binding to the nucleotide sequence at the 3' end of the determined site.

Among PCR applications, nested PCR is a technique that can increase the specificity and sensitivity of PCR amplification, and it is typically used to amplify low-purity DNA or RNA. In this method, as continuous PCR by two types of primers, the first polymerase chain reaction is performed by using the primer of a target gene, and the second polymerase chain reaction is performed by using a second primer that binds to the first PCR product. In particular, the nested PCR method is a method that amplifies with a first amplification primer and then amplifies by adding a second primer again, and this method is often used when increased sensitivity or special purpose amplification is required. However, since nested PCR usually requires using two different tubes, it is inconvenient and laborious for the experimenter, and there is a problem in that it may cause contamination during handling of the amplification product. The situation is that a PCR method is required, in which contamination from the outside is reduced but the amplification time of the template gene can be reduced and the amount of amplification can be increased compared to conventional PCR, and the yield (PCR YIELD) problem in the general polymerase chain reaction used to detect a point mutation can be resolved (in general, when conducting PCR to detect a point mutation, there is a problem in that the PCR YIELD is reduced by using a low concentration of primers and performing an annealing step at a temperature slightly higher than the temperature of a general annealing step).

### [Disclosure]

### [Technical Problem]

An object of the present invention is to provide a polymerase chain reaction method in which the addition process of an intermediate reactant is not performed and contamination from the outside is reduced.

Another object of the present invention is to provide a polymerase chain reaction method which is capable of shortening the gene amplification time.

Still another object of the present invention is to provide a polymerase chain reaction method that can be easily amplified even when a small amount (low concentration) of a template gene is present.

Still another object of the present invention is a polymerase chain reaction method which is capable of synthesizing a larger amount of product in a faster time by decreasing the concentration of a second reverse primer and increasing the concentration of a third reverse primer, as a solution to the yield (PCR YIELD) problem in a conventional polymerase chain reaction used to detect a point mutation.

Still another object of the present invention is to provide a kit for a polymerase chain reaction in which contamination from the outside is reduced because the process of adding an intermediate reactant is not performed.

### [Technical Solution]

In order to solve the above-described problems, the present invention provides a method for performing a sequential polymerase chain reaction, including preparing a composition including a template gene, DNA polymerase, dNTP, a first forward primer, a first reverse primer, a second reverse primer and a third reverse primer in one container; a first polymerase chain reaction step; and a second polymerase chain reaction step.

According to an exemplary embodiment of the present invention, the first polymerase chain reaction step may include a) a first denaturation step of unwinding the double helix of the template gene; b) a first annealing step of binding the first forward primer and the first reverse primer to the template gene according to step a); and c) synthesizing a first product by replicating the template gene by the first forward primer and the second reverse primer bound in step b), and wherein the second polymerase chain reaction step may include d) a second denaturation step of unwinding the double helix of the first product; e) a second annealing step of binding the first forward primer and the first reverse primer to the first product according to step d); f) synthesizing a second product by replicating the first product by the first forward primer and the second reverse primer bound in step e); and g) synthesizing a final product by binding the first forward primer to the second product to synthesize a third product, and binding the third reverse primer to the third product.

The first polymerase chain reaction and the second polymerase chain reaction of the present invention may be controlled according to temperature, and the performance temperature in step e) may be higher than the performance temperature in step b). According to an exemplary embodiment of the present invention, step e) may be performed at a temperature which is 5°C to 30°C higher than step b). According to another exemplary embodiment of the present invention, when step b) is performed at a temperature of 40°C or higher to below 50°C, step e) may be performed at a temperature of 50°C or higher to 72°C or lower, and when step b) is performed at a temperature of 40°C or higher to below 60°C, step e) may be performed at a temperature of 60°C or higher to 72°C or lower.

According to an exemplary embodiment of the present invention, the second reverse primer may further include an arbitrary gene (adapter gene) sequence in addition to a sequence that binds to the template gene. The second reverse primer may further include an arbitrary gene sequence, and the arbitrary gene sequence or a complementary sequence thereof may be included in the second product or the third product.

Although the arbitrary gene sequence included in the second reverse primer according to the present invention is not particularly limited, it is preferable that it does not bind to the first product because it does not include a gene sequence complementary to the gene sequence of the first product.

According to an exemplary embodiment of the present invention, the composition may further include a probe binding to the third product. A gene sequence of the third product to which the probe binds may be a gene sequence or a partial sequence thereof complementary to an arbitrary gene sequence included in the second reverse primer. In the present invention, by binding to a gene sequence complementary to the arbitrary gene sequence, the probe may confirm the presence of a second product, that is, whether the second polymerase chain reaction proceeds.

According to an exemplary embodiment of the present invention, provided is a composition for a sequential polymerase chain reaction, including a template gene, DNA polymerase, dNTP, a first forward primer, a first reverse primer, a second reverse primer and a third reverse primer.

In addition, the present invention provides a kit for a sequential polymerase chain reaction, including a template gene, DNA polymerase, dNTP, a first forward primer, a first reverse primer, a second reverse primer and a third reverse primer.

### [Advantageous Effects]

In the kit for a sequential polymerase chain reaction and the gene amplification method using the same according to the present invention, a reactant-adding step is omitted unlike conventional nested PCR, and thus, the inflow of contaminants from the outside is low, and the amplification time can be shortened.

The sequential polymerase chain reaction (switching PCR) of the present invention is characterized by having high reproducibility even in the presence of a low concentration of a target gene (the gene to be replicated in the PCR).

For example, in the case of general 10³ copy RT-PCR, reproducible PCR results are shown only when 10² copies of the target gene (target template) are present. On the other hand, the sequential polymerase chain reaction according to the present invention has high reproducibility even when the concentration of the target gene is low by using the second polymerase chain reaction, the second reverse primer and the third reverse primer.

According to the sequential polymerase chain reaction method of the present invention, the concentration of the second reverse primer is lowered, but the concentration of the third reverse primer is increased to synthesize a larger amount of product in a faster time, and thus, there is an advantage that can solve the yield (PCR YIELD) problem in the conventional polymerase chain reaction used to detect a point mutation.

In addition, the kit for a polymerase chain reaction according to the present invention has the advantage of high sensitivity by easily amplifying a small amount (low concentration) of a gene (template).

### [Description of Drawings]

FIG. 1 is a mimetic diagram of the sequential polymerase chain reaction according to an exemplary embodiment of the present invention.

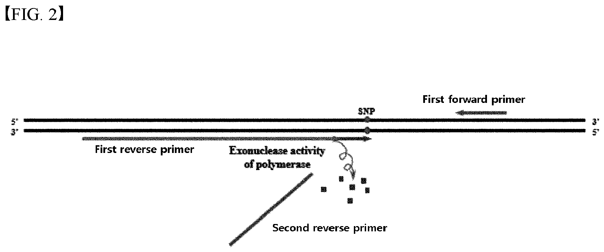

FIG. 2 is a mimetic diagram of the first polymerase chain reaction according to an exemplary embodiment of the present invention.
FIG. 3 is a flowchart of the sequential polymerase chain reaction according to an exemplary embodiment of the present invention.
FIG. 4 is a flowchart of the sequential polymerase chain reaction according to another exemplary embodiment of the present invention.
FIG. 5 is a graph showing the results of amplification of the T790M gene in the sequential polymerase chain reaction according to an exemplary embodiment of the present invention.
FIG. 6 is a graph showing the results of amplification of the T790M gene using a conventional polymerase chain reaction according to an exemplary embodiment.
FIG. 7 is a mimetic diagram of the sequential polymerase chain reaction steps according to another exemplary embodiment of the present invention.
FIG. 8 is a graph showing the results of amplification of the T790M gene using a conventional polymerase chain reaction according to another exemplary embodiment of the present invention.
FIG. 9 is a mimetic diagram of the sequential polymerase chain reaction steps according to another exemplary embodiment of the present invention.
FIG. 10 is a graph showing the results of amplification of the T790M gene using a conventional polymerase chain reaction according to another exemplary embodiment of the present invention.
FIG. 11 is an electrophoretic image of the sequential polymerase chain reaction products according to an exemplary embodiment of the present invention.

### [Modes of the Invention]

Hereinafter, preferred exemplary embodiments of the present invention will be described in detail. The advantages and features of the present invention will be apparent with reference to the exemplary embodiments described below for achieving the same. However, the present invention is not limited to the exemplary embodiments to be disclosed below, but may be implemented in a variety of different forms. However, these exemplary embodiments are provided to make the disclosure of the present invention complete, and to fully inform the scope of the invention to those skilled in the art to which the present invention pertains, and the present invention is defined by the scope of the claims. The same reference numerals refer to the same constitutional elements throughout the specification.

Unless otherwise defined, all terms (including technical and scientific terms) used in the present specification may be used as meanings that can be commonly understood by those of ordinary skill in the art to which the present invention pertains. In addition, terms that are defined in a commonly used dictionary are not interpreted ideally or excessively unless explicitly defined specifically. The terms used in the present specification are for describing the exemplary embodiments and are not intended to limit the present invention. In the present specification, the singular form also includes the plural form unless specifically stated in the phrase.

As used herein, the term ' polymerase chain reaction (PCR)' is a method of amplifying a specific template gene to be detected and as modifications of the polymerase chain reaction, it includes reverse transcriptase polymerase chain reaction (RT-PCR) in which complementary DNA is synthesized by using reverse transcriptase for RNA and performs a polymerase chain reaction by using the same as a template, real-time polymerase chain reaction that simultaneously detects an amplified product while amplifying DNA using fluorescent materials and the like.

Unless otherwise specified herein, the sequential polymerase chain reaction of the present invention includes a process carried out by a method commonly used in the art.

For example, the enzymes used in the sequential polymerase chain reaction of the present invention may include DNA Taq-polymerase having exonuclease activity, which may be used in the same manner in a first polymerase chain reaction and a second polymerase chain reaction.

As used herein, the term 'primer' is a nucleic acid sequence having a short free 3' hydroxyl group to be replicated and may form base pairs with a complementary template, and it refers to a short nucleic acid sequence that functions as a starting point for copying the template gene. The length and sequence of a primer should allow the synthesis of an extension product to begin. The specific length and sequence of a primer will depend on the complexity of a DNA or RNA target required as well as the conditions of use of the primer, such as temperature and ionic strength.

As used herein, the term 'template gene' may include double-stranded DNA, single-stranded RNA, cDNA or miRNA, and most preferably, the template gene is in the form of DNA.

As used herein, the term 'product' refers to a gene synthesized according to a polymerase chain reaction.

As used herein, the term 'degradation' of a probe or primer may mean that the structure of a probe or primer is physically or chemically modified or destroyed.

As with the commonly known polymerase chain reaction (PCR) technique, one PCR step consists of three steps. After passing through a thermal denaturation process (denaturation step) that separates genes using heat, the temperature is lowered to allow the primer to bind to the end of a sequence to be amplified (annealing step), and the heat is slightly raised to perform a polymerization reaction (polymerization or extension, gene synthesis step) that synthesizes the gene, The thermal denaturation step, the annealing step and the gene synthesis step are sequentially performed, and it is noted that one cycle is performed when each step is performed once. For example, in the case of the first polymerase chain reaction of the present invention, when a denaturation step is performed at 95°C for 15 seconds, an annealing step is performed at 65°C for 30 seconds, and a gene synthesis step is performed at 72°C for 30 seconds, it is noted that in this case, the first polymerase chain reaction is performed for 1 cycle (times).

As used herein, the term 'sequential polymerase chain reaction' may include preparing a composition including a template gene, DNA polymerase, dNTP, a first forward primer, a first reverse primer, a second reverse primer and a third reverse primer in one container; a first polymerase chain reaction step; and a second polymerase chain reaction step.

The first polymerase chain reaction step of the present invention may include a) a first denaturation step of unwinding the double helix of the template gene; b) a first annealing step of binding the first forward primer and the first reverse primer to the template gene according to step a); and c) synthesizing a first product by replicating the template gene by the first forward primer and the second reverse primer bound in step b). The second polymerase chain reaction step may include d) a second denaturation step of unwinding the double helix of the first product; e) a second annealing step of binding the first forward primer and the first reverse primer to the first product according to step d); f) synthesizing a second product by replicating the first product by the first forward primer and the second reverse primer bound in step e); and g) synthesizing a final product by binding the first forward primer to the second product to synthesize a third product, and binding the third reverse primer to the third product.

As used herein, the term 'container' includes all types of experimental tools capable of causing a polymerase chain reaction. The present invention is characterized in that different polymerase chain reactions are continuously and independently performed in one container.

As used herein, the term 'sequential polymerase chain reaction (hereinafter, referred to as 'switching PCR')' means a reaction in which a first polymerase chain reaction for amplifying a template gene and a second polymerase chain reaction for amplifying the product of the first polymerase chain reaction occur continuously and sequentially, and the initiation of each polymerase chain reaction may be controlled by temperature. The sequential polymerase chain reaction according to the present invention may further proceed to a third or fourth polymerase chain reaction or subsequent steps.

In the sequential polymerase chain reaction of the present invention, a separate reactant-adding step is excluded between the first polymerase chain reaction and the second polymerase chain reaction, and the first polymerase chain reaction and the second polymerase chain reaction may be performed continuously or independently in one container. Unlike the conventional nested PCR reaction, in the 'sequential polymerase chain reaction' according to the present invention, the intermediate step of adding reactants is omitted, thereby shortening the gene amplification time and preventing contamination from foreign substances.

The first polymerase chain reaction or the second polymerase chain reaction may be performed in several cycles, and the nested PCR technique may be used as a part of the sequential polymerase chain reaction of the present invention. The present invention may amplify the gene in a short period of time even if the template gene (template) is present at a low concentration by passing through multiple PCR steps.

Similar to the conventional polymerase chain reaction (PCR), the main purpose of the present invention is to amplify the amount of a template gene, and the first polymerase chain reaction and the second polymerase chain reaction of the 'sequential polymerase chain reaction' according to the present invention may be performed several times to synthesize a large amount of the final gene (final product). As described above, the genes synthesized in each step of the present invention are used or remain in the subsequent reaction.

As used herein, the term 'forward' or 'reverse' indicating the direction of initiation of the synthesis of primers means that the synthesis direction between each primer is different or identical, and it does not specify the gene synthesis direction of 5'- >3' or 3'->5' according to the replication target gene (e.g., template gene). That is, although the gene synthesis direction of the primers according to the replication target gene may be different, the synthesis direction between the reverse primers is the same and is opposite to that of the forward primer.

For example, when the template gene is double-stranded, the first forward primer and the first reverse primer may each replicate complementary template gene strands (first polymerase chain reaction). Thereafter, the first forward primer may bind to a second product synthesized by the first reverse primer, and the second reverse primer may bind to the second product synthesized by the first forward primer to replicate the gene strand (second polymerase chain reaction). The first forward primer may bind to the second product synthesized by the second reverse primer to initiate the synthesis of a third product, and in this case, when an arbitrary gene sequence is further included in the second reverse primer, a gene sequence complementary to the arbitrary gene sequence may be further synthesized in the third product. The third reverse primer may bind to the third product synthesized by the first forward primer to initiate the synthesis of a final product. In this case, if a probe and the third reverse primer bind to the 'sequence complementary to the arbitrary gene sequence' included in the third product, the probe separates from the third product and emits light according to the initiation of gene synthesis of the third reverse primer. In this case, since the light emission by the probe is confirmed only when the third product is synthesized, it can be seen that the second polymerase chain reaction has been performed.

As can be seen from the foregoing, the first product or the second product of the present invention may be composed of double strands, and each primer of the present invention binds to a gene sequence complementary to each gene, and the subsequent reaction proceeds.

As used herein, the terms 'first forward primer' and 'first reverse primer' refer to primers that bind to a template gene and initiate the replication of a template gene. A first product is synthesized according to the initiation of template gene replication of the first forward primer and the first reverse primer. In the present invention, the 'first reverse primer' is designed to bind only to the template gene, but the 'first forward primer' binds not only to the template gene but also binds to the first product, the second product or the third product to initiate replication.

As used herein, the term 'second reverse primer' refers to a primer that binds to the first product, and the second reverse primer may also bind to the template strand, but is designed such that it does not participate in the first polymerase chain reaction by controlling the activation temperature and the temperature of the first annealing step. When the template gene is DNA, since the first product is also double-stranded, the first forward primer binds to the complementary strand of the first product gene strand, to which the second reverse primer binds, to initiate the replication of the first product gene. In this case, the second reverse primer may further include an arbitrary gene (adapter gene) sequence which is not included in the first product or template gene, and the arbitrary gene may be located at the 5' end of the second reverse primer. Accordingly, the second product synthesized by the second reverse primer may include the arbitrary gene sequence which is not included in the template gene.

The first forward primer may bind to the second product synthesized by the second reverse primer to initiate the synthesis of the third product.

As used herein, the term 'third reverse primer' refers to a primer that binds to the third product. The third reverse primer may bind to 'a gene sequence complementary to the arbitrary gene sequence' present in the 'third product.' According to the present invention, the third reverse primer is not involved in the first polymerase chain reaction, because it binds to the 'gene sequence complementary to the arbitrary gene sequence.'

When the polymerase chain reaction (PCR) is performed, it is common to reduce the concentration of primers used in order to detect a point mutation of a template gene, but in this case, there is a problem in that the amount (yield) of the final product is reduced. In order to solve this problem, the present invention uses a second reverse primer including an arbitrary gene and a third reverse primer. In the present invention, the second reverse primer (preferably at the 5' end) may include an arbitrary gene (adapter gene) and initiates the second polymerase chain reaction by using the first product as a template. According to the present invention, the first forward primer may bind to the second product synthesized by the second reverse primer, whereby the third product including the 'gene complementary to the arbitrary gene (adapter gene)' is synthesized. The third reverse primer binds to 'a gene complementary to an arbitrary gene (adapter gene)' included in the third product to initiate the synthesis of a final product, and since a high concentration may be used for the concentration of the third reverse primer regardless of a point mutation, more products may be produced. That is, in order to solve the yield (PCR YIELD) problem in a conventional polymerase chain reaction for detecting a point mutation (in general, when conducting PCR to detect a point mutation, there is a problem in that the PCR YIELD is reduced by using a low concentration of primers and performing an annealing step at a temperature slightly higher than the temperature of a general annealing step), the concentration of the second reverse primer used may be lowered, but the concentration of the third reverse primer may be increased, and thus, a larger amount of product may be synthesized in a short period of time.

As used herein, the term 'design' of a primer means preparing a primer such that the primer can be activated or blocked under specific conditions (e.g., temperature), and the primer design method commonly used in the art may be used. The design method includes a method of controlling the length (mer) or sequence of a primer, but is not limited thereto.

As used herein, the term 'blocking' may mean that the primer is not annealed to the target gene (gene (template) to be replicated in the corresponding PCR).

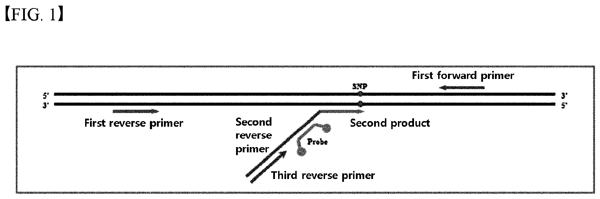

FIG. 1 is a mimetic diagram of the sequential polymerase chain reaction (switching PCR) according to an exemplary embodiment of the present invention. Referring to FIG. 1, a first primer may bind to a template gene and an induction primer may bind to a first product to synthesize a second product. FIG. 1 only expresses the first polymerase chain reaction and the second polymerase chain reaction step in one mimetic diagram, and the first polymerase chain reaction and the second polymerase chain reaction do not proceed simultaneously.

In the present invention, each annealing step of the first polymerase chain reaction and the second polymerase chain reaction may have different temperatures, and preferably, the annealing step temperature of the second polymerase chain reaction is higher than the annealing temperature of the first polymerase chain reaction. For example, the annealing step of the second polymerase chain reaction may be performed at a temperature 5°C to 30°C higher than the annealing step of the first polymerase chain reaction. According to the present invention, the temperature at which each primer is activated may be different from each other. In this case, the activation temperature of the first forward primer is preferably designed in the widest range, and the activation temperatures of the second reverse primer and the third reverse primer are preferably designed to be higher than the activation temperature of the first reverse primer. For example, the activation temperature of the second reverse primer or the third reverse primer may be designed to be 5°C to 30°C higher than the activation temperature of the first reverse primer.

The annealing step of the first polymerase chain reaction according to the present invention may be performed at a temperature 5°C to 30°C lower than the annealing step of the second polymerase chain reaction, and exonuclease is activated by the initiation of gene synthesis of the first reverse primer. That is, in the first polymerase chain reaction, only the first product by the first forward primer and the first reverse primer may be synthesized. The annealing step of the second polymerase chain reaction may be performed at a temperature which is 5°C to 30°C higher than the annealing step of the first polymerase chain reaction, and due to the relatively high temperature, the reaction by the first reverse primer does not proceed. On the other hand, the second reverse primer may bind to the first product such that only the second polymerase chain reaction proceeds.

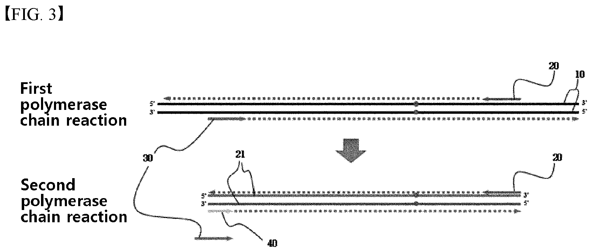

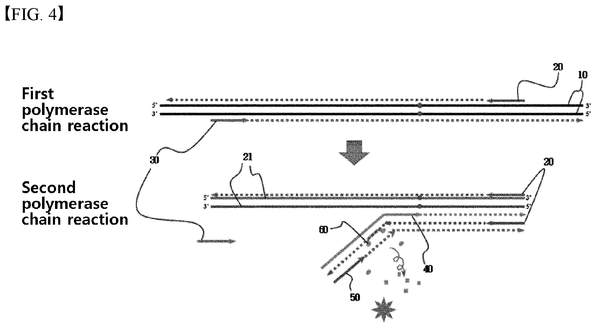

Specifically, FIGS. 3 and 4 show mimetic diagrams of the sequential polymerase chain reaction according to the present invention.

Referring to FIG. 3, a first forward primer 20 and a first reverse primer 30 bind to a template gene strand 10 to initiate gene synthesis in the first polymerase chain reaction. A first product 21 is synthesized according to the first polymerase chain reaction, and the first forward primer 20 and the second reverse primer 40 bind to the first product again to perform a second polymerase chain reaction. Referring to FIG. 3, the first reverse primer 30 is activated in the first polymerase chain reaction, but is not activated in the second polymerase chain reaction.

Referring to FIG. 4, the first forward primer 20 and the second reverse primer 40 may bind to a first product according to the second polymerase chain reaction to synthesize a gene. In this case, the second reverse primer 40 may further include an arbitrary gene sequence in addition to the gene sequence binding to the first product (or the gene sequence binding to the template gene). The second product synthesized by the second reverse primer further includes an arbitrary gene sequence in addition to the template gene. The probe of the present invention may bind to an arbitrary gene synthesized in the second product. When the third reverse primer 50 binds to the second product to initiate gene synthesis, the probe may display a specific signal away from the second product, and it can be seen that the second polymerase chain reaction has been performed by identifying the corresponding signal.

According to an exemplary embodiment of the present invention, the first forward primer, the first reverse primer, the second reverse primer, the third reverse primer and the probe may consist of sequences as shown in Table 1 below.

**[Table 1]**

| | Sequence Information (5'→3') |
|---|---|
| First forward primer | GGGAGCCAATATTGTCTTTGTGTTC |
| First reverse primer | GTGCCGCCTGCTG |
| Second reverse primer (Example 1) | CTCCACCGTGCAGCTCATCA |
| Second reverse primer (Example 2) | TAGGCGGAATCGGTAGTAATCAA CAT CAGTCTGATAAGCTATCGG TAC CTCCACCGTGCAGCTCATCAC |
| Third reverse primer | TAGGCGGAATCGGTAGTAATCAA |
| Probe | /56-FAM/ CAG TCT GAT /ZEN/ AAG CTA TCG G /3IABkFQ/ |

Hereinafter, the present invention will be described in more detail through examples. These examples are only for illustrating the present invention in more detail, and it will be apparent to those skilled in the art that the scope of the present invention is not limited by these examples according to the gist of the present invention.

### [Example 1]

### <1-1> Switching PCR reaction using template of standard concentration

10 µL of qPCR Master mix was added to a PCR tube. Then, 1 µL of Template DNA (T790M) was added, and 1 µL of each of the first forward primer and the first reverse primer at a concentration of 10 µM, 1 µL of the second reverse primer at a concentration of 10 µM, 1 µL of the third reverse primer and 0.5 µL of the probe were added. After adding 7 µL of distilled water to the tube, pipetting was performed (in this case, the prepared kit is referred to as Experimental Group 1). Thereafter, the tube was placed into the well of a PCR machine (Bio-Rad CFX96^{™}), and the temperature was adjusted as shown in Table 2 below to perform the first polymerase chain reaction 20 times and the second polymerase chain reaction for 40 times.

**[Table 2]**

| Temperature Conditions | Time | Number of Cycles |
|---|---|---|
| 95°C | 15 minutes | |
| 95°C | 15 seconds | First polymerase chain reaction 20 times |
| 65°C | 30 seconds | |
| 72°C | 30 seconds | |
| 95°C | 15 seconds | Second polymerase chain reaction 40 times |
| 71°C | 30 seconds | |
| 72°C | 30 seconds | |

### <1-2> Switching PCR reaction using diluted template

Experimental groups were prepared by setting the concentration of Template DNA (T790M) to 1/2 µL (Experimental Group 2), 1/4 µL (Experimental Group 3), 1/6 µL (Experimental Group 4), 1/8 µL (Experimental Group 5) and 1/10 µL (Experimental Group 6), and experiments were performed under the same rest of conditions as in Experimental Example 1-1.

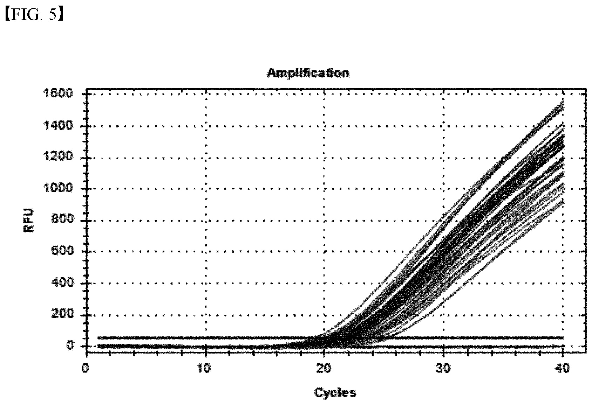

Result values according to Examples 1-1 and 1-2 are shown in Tables 3 and 4 below. FIG. 5 is a graph showing the results of amplification of the T790M gene in the sequential polymerase chain reaction (switching PCR) according to Examples 1-1 and 1-2 of the present invention.

**[Table 3]**

| Gene Type | Concentration | Cq (Number of Cycles) | Cq Average | Cq Standard Deviation |
|---|---|---|---|---|
| PC (T790M Mutant standard) | 1%M (3 COPY) | 19.69 | 19.69 | 0.38 |
| | | 20.23 | | |
| | | 19.12 | | |
| | | 19.73 | | |
| | 1/2%M | 21.22 | 21.43 | 0.64 |
| | | 21.05 | | |
| | | 20.47 | | |
| | | 21.47 | | |
| | | 21.51 | | |
| | | 21.78 | | |
| | | 21.13 | | |
| | | 20.92 | | |
| | | 20.21 | | |
| | | 21.06 | | |
| | | 21.99 | | |
| | | 20.45 | | |
| | | 22.59 | | |
| | | 21.60 | | |
| | | 22.17 | | |
| | | 21.75 | | |
| | | 21.87 | | |
| | | 22.15 | | |
| | | 21.79 | | |

**[Table 4]**

| Gene Type | Concentration | Cq (Number of Cycles) | Cq Average | Cq Standard Deviation |
|---|---|---|---|---|
| PC (T790M Mutant standard) | 1/4%M | 23.42 | 23.15 | 1.07 |
| | | 22.16 | | |
| | | 22.26 | | |
| | | 23.01 | | |
| | | 21.07 | | |
| | | 23.51 | | |
| | | 22.88 | | |
| | | 23.68 | | |
| | | 21.97 | | |
| | | 23.68 | | |
| | | 21.97 | | |
| | | 23.27 | | |
| | | 24.07 | | |
| | | 23.05 | | |
| | | 23.31 | | |
| | | 25.39 | | |
| | | 23.10 | | |
| | | 22.09 | | |
| | | 23.41 | | |
| | | 24.34 | | |
| | | 25.18 | | |

### <1-3> Standard PCR reaction using template of reference concentration

10 µL of qPCR Master mix was added to a PCR tube. Thereafter, 1 µL of Template DNA (T790M) was added, and 0.5 µL of each of the first forward primer and the first reverse primer at a concentration of 10 uM was added. After adding 7 µL of distilled water to the tube, pipetting was performed (in this case, the prepared kit is referred to as Control Group 1). Thereafter, the tube was placed into the well of a PCR machine (Bio-Rad CFX96^{™}), and the temperature was controlled as shown in Table 5 below to perform PCR for 60 times.

**[Table 5]**

| Temperature Conditions | Times | Number of Cycles |
|---|---|---|
| 95°C | 15 minutes | |
| 95°C | 15 seconds | PCR 60 times |
| 71°C | 30 seconds | |
| 72°C | 30 seconds | |

### <1-4> Standard PCR reaction using diluted template

Experimental groups were prepared by setting the concentration of Template DNA (T790M) to 1/2 µL (Experimental Group 2), 1/4 µL (Experimental Group 3), 1/6 µL (Experimental Group 4), 1/8 µL (Experimental Group 5) and 1/10 µL (Experimental Group 6), and experiments were performed under the same rest of conditions as in Experimental Example 2-1.

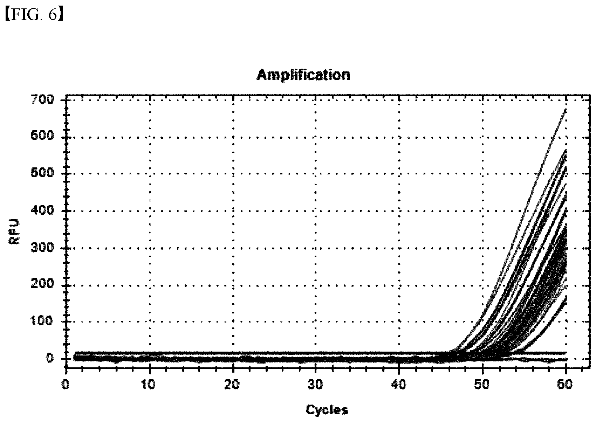

Result values according to Examples 1-3 and 1-4 are shown in Tables 6 and 7 below. FIG. 6 is a graph showing the results of amplification of the T790M gene according to Examples 1-3 and 1-4 of the present invention.

**[Table 6]**

| Gene Type | Concentration | Cq (Number of Cycles) | Cq Average | Cq Standard Deviation |
|---|---|---|---|---|
| PC (T790M Mutant standard) | 1%M (3 COPY) | 46.36 | 46.13 | 0.54 |
| | | 45.60 | | |
| | | 45.75 | | |
| | | 46.79 | | |
| | 1/2%M | 51.06 | 49.58 | 1.87 |
| | | 48.25 | | |
| | | 50.83 | | |
| | | 49.34 | | |
| | | 49.53 | | |
| | | 49.94 | | |
| | | 46.98 | | |
| | | 49.78 | | |
| | | 49.96 | | |
| | | 48.02 | | |
| | | 46.97 | | |
| | | 50.99 | | |
| | | 50.57 | | |
| | | 51.28 | | |
| | | 46.23 | | |
| | | 53.65 | | |
| | | 48.14 | | |
| | | 51.00 | | |

**[Table 7]**

| Gene Type | Concentration | Cq (Number of Cycles) | Cq Average | Cq Standard Deviation |
|---|---|---|---|---|
| PC (T790M Mutant standard) | 1/4%M | 52.59 | 51.51 | 1.32 |
| | | 51.53 | | |
| | | 51.16 | | |
| | | 51.66 | | |
| | | 51.22 | | |
| | | 51.81 | | |
| | | 51.35 | | |
| | | 52.10 | | |
| | | 48.10 | | |
| | | 50.26 | | |
| | | 50.99 | | |
| | | 50.31 | | |
| | | 50.58 | | |
| | | 53.42 | | |
| | | 50.96 | | |
| | | 52.23 | | |
| | | 53.52 | | |
| | | 53.35 | | |

Referring to FIG. 5, as a result of the sequential polymerase chain reactions (switching PCR) according to Examples 1-1 and 1-2, the T790M gene showed Cq or Ct values of 18 to 24 cycles, and referring to FIG. 6, when the T790M gene was amplified according to the standard PCR method according to Examples 1-3 and 1-4, it was amplified in 48 to 50 cycles. These are the same as the contents of Tables 3, 4, 6 and 7. Considering that the sequential polymerase chain reaction (switching PCR) according to the present invention is amplified in the second polymerase chain reaction, as a result of the sequential polymerase chain reactions (switching PCR) according to Examples 1-1 and 1-2, it can be seen that the T790M gene had Cq or Ct values in 38 to 44 cycles (the first polymerase chain reaction occurs in 20 cycles). In all of Examples 1-1 to 1-4, the Cq or Ct values increased as the concentration of the T790M gene as a template was lowered, and the amplification cycle values of Examples 1-1 and 1-2 were lower than the values of Examples 1-3 and 1-4 regardless of the concentration. Therefore, it can be seen that in the sequential polymerase chain reaction (switching PCR) method according to the present invention, the first polymerase chain reaction and the second polymerase chain reaction can be independently performed in one experimental apparatus.

### [Example 2]

### <2-1> Switching PCR reaction using template at reference concentration

PCR was performed under the same conditions as in Example 1-1.

### <2-2> Switching PCR reaction using diluted template

PCR was performed under the same conditions as in Example 1-2.

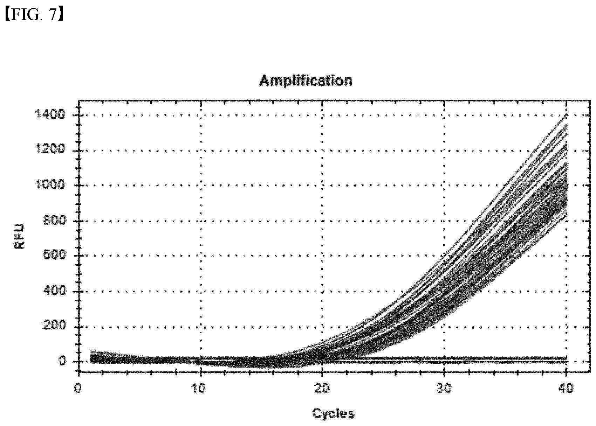

Result values according to Examples 2-1 and 2-2 are shown in Tables 8 and 9 below. FIG. 7 is a graph showing the results of amplification of the T790M gene in the sequential polymerase chain reactions (switching PCR) according to Examples 2-1 and 2-2 of the present invention.

**[Table 8]**

| Gene Type | Concentration | Cq (Number of Cycles) | Cq Average | Cq Standard Deviation |
|---|---|---|---|---|
| PC (T790M Mutant standard) | 1/4% M | 17.59 | 19.99 | 1.67 |
| | | 19.95 | | |
| | | 21.56 | | |
| | | 20.85 | | |
| | 1/6% M | 19.10 | 20.66 | 1.40 |
| | | 20.02 | | |
| | | 19.34 | | |
| | | 19.52 | | |
| | | 19.93 | | |
| | | 21.31 | | |
| | | 23.11 | | |
| | | 19.53 | | |
| | | 19.66 | | |
| | | 21.86 | | |
| | | 22.47 | | |
| | | 23.18 | | |
| | | 20.26 | | |
| | | 19.74 | | |
| | | 18.55 | | |
| | | 22.15 | | |
| | | 19.88 | | |
| | | 21.97 | | |
| | | 21.42 | | |
| | | 20.17 | | |

**[Table 9]**

| Gene Type | Concentration | Cq (Number of Cycles) | Cq Average | Cq Standard Deviation |
|---|---|---|---|---|
| PC (T790M Mutant standard) | 1/8% M | 23.17 | 22.97 | 0.69 |
| | | 22.22 | | |
| | | 21.52 | | |
| | | 22.69 | | |
| | | 23.26 | | |
| | | 23.34 | | |
| | | 23.30 | | |
| | | 23.48 | | |
| | | 23.89 | | |
| | | 23.48 | | |
| | | 23.97 | | |
| | | 22.46 | | |
| | | 22.37 | | |
| | | 23.91 | | |
| | | 23.87 | | |
| | | 23.13 | | |
| | | 23.45 | | |
| | | 22.49 | | |
| | | 23.51 | | |
| | | 24.14 | | |

### <2-3> Standard PCR reaction using template at reference concentration

PCR was performed under the same conditions as in Example 1-3.

### <2-4> Standard PCR reaction using diluted template

PCR was performed under the same conditions as in Examples 1-4.

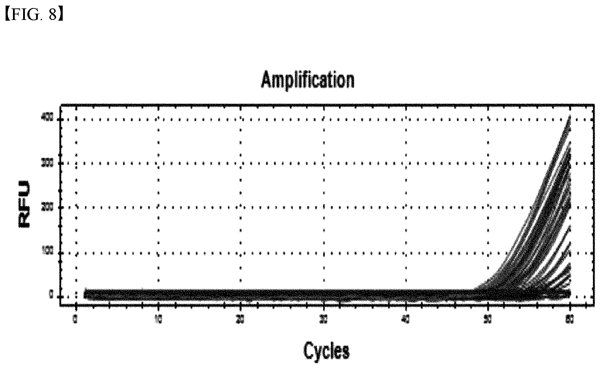

Result values according to Examples 2-3 and 2-4 are shown in Tables 10 and 11 below. FIG. 8 is a graph showing the results of amplification of the T790M gene according to Examples 2-3 and 2-4 of the present invention.

**[Table 10]**

| Gene Type | Concentration | Cq (Number of Cycles) | Cq Average | Cq Standard Deviation |
|---|---|---|---|---|
| PC (T790M Mutant standard) | 1/4%M | 51.16 | 51.04 | 1.31 |
| | | 52.26 | | |
| | | 49.19 | | |
| | | 51.53 | | |
| | 1/6% M | 54.55 | 50.22 | 2.08 |
| | | 52.15 | | |
| | | 55.60 | | |
| | | 48.52 | | |
| | | 48.80 | | |
| | | 50.73 | | |
| | | 49.31 | | |
| | | 50.34 | | |
| | | 48.27 | | |
| | | 49.07 | | |
| | | 49.55 | | |
| | | 50.57 | | |
| | | 50.70 | | |
| | | 48.60 | | |
| | | 48.13 | | |
| | | 47.81 | | |
| | | 51.44 | | |
| | | 51.51 | | |
| | | 48.52 | | |
| | | 50.30 | | |

**[Table 11]**

| Gene Type | Concentration | Cq (Number of Cycles) | Cq Average | Cq Standard Deviation |
|---|---|---|---|---|
| PC (T790M Mutant standard) | 1/8% M | 55.68 | 53.09 | 3.05 |
| | | 50.66 | | |
| | | 53.22 | | |
| | | 49.04 | | |
| | | 54.04 | | |
| | | 50.89 | | |
| | | 50.25 | | |
| | | 49.38 | | |
| | | 50.72 | | |
| | | 49.55 | | |
| | | 52.74 | | |
| | | 51.72 | | |
| | | 54.54 | | |
| | | 58.93 | | |
| | | 53.63 | | |
| | | 56.08 | | |
| | | 58.00 | | |
| | | 56.60 | | |

Referring to FIG. 7, as a result of the sequential polymerase chain reactions (switching PCR) according to Examples 2-1 and 2-2, the T790M gene showed Cq or Ct values of 18 to 24 cycles, and referring to FIG. 8, when the T790M gene was amplified according to the standard PCR method according to Examples 2-3 and 2-4, Cq or Ct values were shown in 48 to 60 cycles. These are the same as the contents of Tables 8, 9, 10 and 11. Considering that the sequential polymerase chain reaction (switching PCR) according to the present invention is amplified in the second polymerase chain reaction, as a result of the sequential polymerase chain reactions (switching PCR) according to Examples 2-1 and 2-2, it can be seen that the T790M gene showed Cq or Ct values in 38 to 44 cycles (the first polymerase chain reaction occurs in 20 cycles). In all of Examples 2-1 to 2-4, the Cq or Ct values increased as the concentration of the T790M gene as a template was lowered, and the Cq or Ct values of Examples 2-1 and 2-2 were lower than the values of Examples 2-3 and 2-4 regardless of the concentration. Therefore, it can be seen that the sequential polymerase chain reaction (switching PCR) method according to the present invention may increase the yield of the gene polymerase chain reaction (PCR) and obtain a highly reproducible polymerase chain reaction result even in the presence of a very low concentration of gene.

### [Example 3]

### <3-1> Switching PCR reaction using template at reference concentration

PCR was performed under the same conditions as in Example 1-1.

### <3-2> Switching PCR reaction using diluted template

PCR was performed under the same conditions as in Example 1-2.

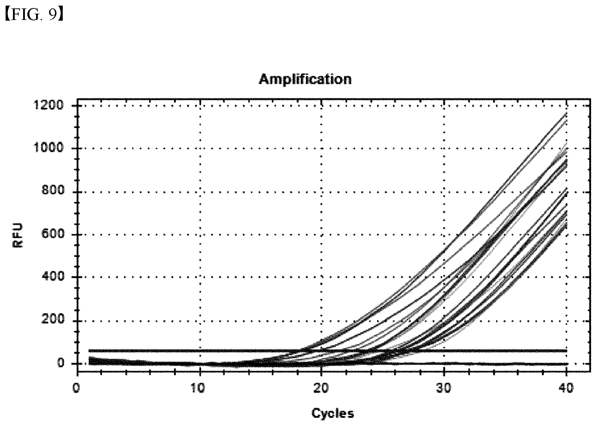

Result values according to Examples 3-1 and 3-2 are shown in Table 12 below. FIG. 9 is a graph showing the results of amplification of the T790M gene in the sequential polymerase chain reactions (switching PCR) according to Examples 3-1 and 3-2 of the present invention.

**[Table 12]**

| Gene Type | Concentration | Cq (Number of Cycles) | Cq Average | Cq Standard Deviation |
|---|---|---|---|---|
| PC (T790M Mutant standard) | 1/2% M | 17.87 | 17.93 | 0.08 |
| | | 17.99 | | |
| | 1/4% M | 18.12 | 18.76 | 0.90 |
| | | 19.39 | | |
| | 1/6% M | 22.22 | 21.69 | 0.75 |
| | | 21.16 | | |
| | 1/8% M | 23.73 | 25.11 | 2.01 |
| | | 28.07 | | |
| | | 24.01 | | |
| | | 24.65 | | |
| | 1/10% M | 26.65 | 25.89 | 1.31 |
| | | 23.84 | | |
| | | 27.14 | | |
| | | 25.49 | | |
| | | 23.44 | | |
| | | 26.05 | | |
| | | 27.03 | | |
| | | 26.11 | | |
| | | 25.94 | | |
| | | 27.18 | | |

### <3-3> Standard PCR reaction using template at reference concentration

PCR was performed under the same conditions as in Example 1-3.

### <3-4> Standard PCR reaction using diluted template

PCR was performed under the same conditions as in Examples 1-4.

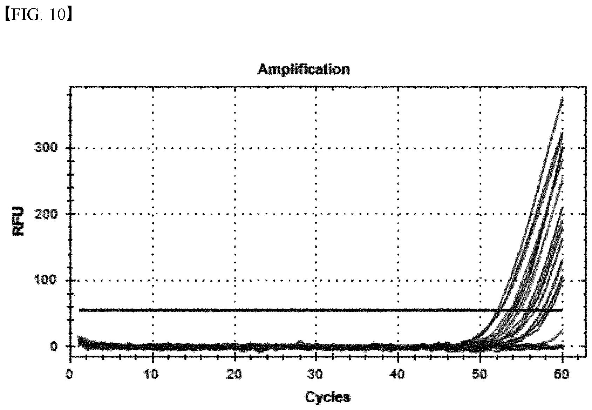

Result values according to Examples 3-3 and 3-4 are shown in Table 13 below. FIG. 10 is a graph showing the results of amplification of the T790M gene in the sequential polymerase chain reactions (switching PCR) according to Examples 3-3 and 3-4 of the present invention.

**[Table 13]**

| Gene Type | Concentration | Cq (Number of Cycles) | Cq Average | Cq Standard Deviation |
|---|---|---|---|---|
| PC (T790M Mutant standard) | 1/2% M | 55.95 | 54.20 | 2.48 |
| | | 52.44 | | |
| | 1/4% M | 52.11 | 52.26 | 0.20 |
| | | 52.40 | | |
| | 1/6% M | 54.60 | 54.09 | 0.71 |
| | | 53.59 | | |
| | 1/8% M | - | 56.84 | 1.72 |
| | | 58.19 | | |
| | | 54.90 | | |
| | | 57.43 | | |
| | 1/10% M | 53.89 | 56.35 | 1.72 |
| | | 56.70 | | |
| | | 57.61 | | |
| | | - | | |
| | | 54.18 | | |
| | | 55.64 | | |
| | | 56.17 | | |
| | | 58.49 | | |
| | | - | | |
| | | 58.11 | | |

Referring to FIG. 9, as a result of the sequential polymerase chain reactions (switching PCR) according to Examples 3-1 and 3-2, the T790M gene showed Cq or Ct values in 18 to 24 cycles, and referring to FIG. 10, when the T790M gene was amplified according to the standard PCR method according to Examples 3-3 and 3-4, it had Cq or Ct values in 48 to 60 cycles. These are the same as the contents of Tables 12 and 13. Considering that the sequential polymerase chain reaction (switching PCR) according to the present invention is amplified even in the second polymerase chain reaction, as a result of the sequential polymerase chain reactions (switching PCR) according to Examples 3-1 and 3-2, it can be seen that the T790M gene showed Cq or Ct values in 38 to 44 cycles (the first polymerase chain reaction occurs in 20 cycles). In all of Examples 3-1 to 3-4, the Cq or Ct values increased as the concentration of the T790M gene as a template was lowered, and the Cq or Ct values of Examples 3-1 and 3-2 were lower than the values of Examples 3-3 and 3-4 regardless of the concentration. Therefore, it can be seen that the sequential polymerase chain reaction (switching PCR) method according to the present invention may decrease the amplification time, increase the yield of the gene polymerase chain reaction (PCR) and obtain a highly reproducible polymerase chain reaction result even in the presence of a very low concentration of gene.

In addition, the sequential polymerase chain reaction (switching PCR) in Example 3-2 of the present invention showed a 100% detection rate at both concentrations of 1/8% (Experimental Group 5) and 1/10% (Experimental Group 6), and in the case of the standard PCR of Example 3-4, it showed a detection rate of 75% at a concentration of 1/8% and a detection rate of 80% at a concentration of 1/10%, and thus, it can be seen that detection rates of below 95% were showed at both concentrations. That is, in the sequential polymerase chain reaction (Switching PCR) according to the present invention, amplification occurs easily even when the concentration of the template gene is low, and thus, it can be seen that the sequential polymerase chain reaction (switching PCR) using the kit according to the present invention has a higher sensitivity.

### [Example 4]

### <4-1> Standard PCR reaction using template at reference concentration

10 µL of qPCR Master mix was added to a PCR tube. Thereafter, 100 fg/µL of Template DNA (T790M) was added, and 0.5 µL of each of the first forward primer and the first reverse primer at a concentration of 10 µM, 1 µL of the second reverse primer at a concentration of 10 µM, 1 µL of the third reverse primer at a concentration of 10 µM and 0.5 µL of a probe were added. After adding 7 µL of distilled water to the tube, pipetting was performed. Thereafter, the tube was placed into the well of a PCR machine (Bio-Rad CFX96^{™}), and the temperature was controlled as shown in Table 14 below to perform 40 cycles of PCR.

**[Table 14]**

| Temperature Conditions | Time | Number of Cycles |
|---|---|---|
| 95°C | 15 minutes | |
| 95°C | 15 seconds | PCR 40 times (cycle) |
| 63°C | 30 seconds | |
| 72°C | 30 seconds | |

### <4-2> Standard PCR reaction using template at reference concentration

The temperature was controlled as shown in Table 15, and PCR was performed under the same conditions as in Example 4-1 for other experimental conditions.

**[Table 15]**

| Temperature Conditions | Time | Number of Cycles |
|---|---|---|
| 95°C | 15 minutes | |
| 95°C | 15 seconds | PCR 40 times (cycle) |
| 71°C | 30 seconds | |
| 72°C | 30 seconds | |

### <4-3> Standard PCR reaction using template at reference concentration

The first polymerase chain reaction and the second polymerase chain reaction were performed at the temperatures as shown in Table 16, and PCR was performed under the same conditions as in Example 4-1 for other experimental conditions.

**[Table 16]**

| Temperature Conditions | Time | Number of Cycles |
|---|---|---|
| 95°C | 15 minutes | |
| 95°C | 15 seconds | First polymerase chain reaction 10 times |
| 65°C | 30 seconds | |
| 72°C | 30 seconds | |
| 95°C | 15 seconds | Second polymerase chain reaction 30 times |
| 71°C | 30 seconds | |
| 72°C | 30 seconds | |

### <4-4> Standard PCR reaction using template of standard concentration

The concentration of Template DNA (T790M) was set to 0, and PCR was performed under the same conditions as in Example 4-1 for other experimental conditions.

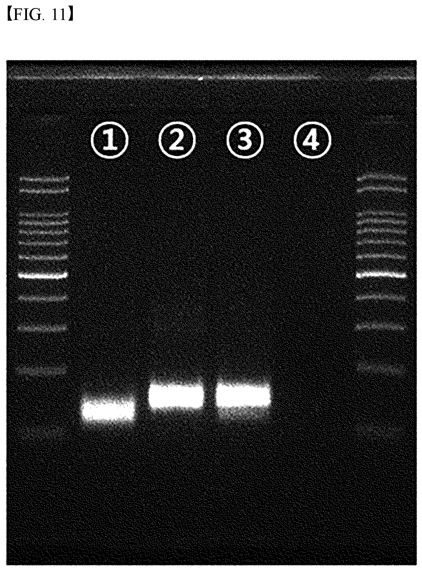

FIG. 11 is an electrophoretic image of the PCR product according to an exemplary embodiment of the present invention. In FIG. 11, ① is a result according to Example 4-1, ② is a result according to Example 4-2, ③ is a result according to Example 4-3 and (4) is a result according to Example 4-4. Referring to FIG. 11, fluorescence was exhibited at 110 bp in ①, a band was exhibited around 131 bp in ②, and no band appeared in (4). Judging ③ based on the above, it can be seen that a weak band appeared at 110 bp and a strong band appeared around 131 bp, and thus, all of products with sizes of 110 bp and 131bp were generated. That is, it can be seen that only one product was synthesized in ① and ②, respectively, and both products of ① and ② were synthesized in ③.

In ① of FIG. 11, since the second reverse primer was not activated in the first polymerase chain reaction, only the first product (110 bp) was formed, and in ②, since the first reverse primer was inactivated due to a high temperature, only the second product (131 bp) was synthesized. On the other hand, in ③ of FIG. 11, since the first polymerase chain reaction was performed for 10 cycles and the second polymerase chain reaction was performed for 30 cycles, it can be confirmed that bands of genes proportional to the performed cycles coexisted (110 bp, 131 bp). According to Example 4-3 of the present invention, by designing primers that can be activated at the annealing temperature of each polymerase chain reaction, the first forward primer and the first reverse primer were activated in the first polymerase chain reaction, and the first forward primer and the second reverse primer were activated in the second polymerase chain reaction, and accordingly, it can be seen that both of the first product and the second product were formed.

### <Explanation of Reference Numerals>

10: Template gene
20: First forward primer
21: First product
30: First reverse primer
40: Second reverse primer
50: Third reverse primer
60: Probe

Although the exemplary embodiments of the present invention have been described above, those of ordinary skill in the art to which the present invention pertains will understand that the present invention may be implemented in other specific forms without changing the technical spirit or essential features thereof. Therefore, it should be understood that the exemplary embodiments described above are illustrative in all respects and not restrictive.

## Claims

1. A method for performing a sequential polymerase chain reaction, comprising preparing a composition comprising a template gene, DNA polymerase, dNTP, a first forward primer, a first reverse primer, a second reverse primer and a third reverse primer in one container; a first polymerase chain reaction step; and a second polymerase chain reaction step,
wherein the first polymerase chain reaction step comprises:
a) a first denaturation step of unwinding the double helix of the template gene;
b) a first annealing step of binding the first forward primer and the first reverse primer to the template gene according to step a); and
c) synthesizing a first product by replicating the template gene by the first forward primer and the second reverse primer bound in step b), and
wherein the second polymerase chain reaction step comprises:
d) a second denaturation step of unwinding the double helix of the first product;
e) a second annealing step of binding the first forward primer and the first reverse primer to the first product according to step d);
f) synthesizing a second product by replicating the first product by the first forward primer and the second reverse primer bound in step e); and
g) synthesizing a final product by binding the first forward primer to the second product to synthesize a third product, and binding the third reverse primer to the third product.

2. The method of claim 1, wherein step e) is performed at a higher temperature than step b), and
wherein the second reverse primer is designed to be activated at a higher temperature than the first reverse primer.

3. The method of claim 1, wherein step e) is performed at a temperature which is 5°C to 30°C higher than step b).

4. The method of claim 1, wherein the first polymerase chain reaction and the second polymerase chain reaction are controlled according to temperature.

5. The method of claim 1, wherein the second reverse primer further comprises an arbitrary gene sequence in addition to a sequence complementary to the template gene.

6. The method of claim 5, wherein the arbitrary gene sequence and the complementary gene sequence are included in the second product, and
wherein the third reverse primer binds to the arbitrary gene sequence and the complementary gene sequence included in the second product to synthesize a final product.

7. The method of claim 1, wherein the composition further comprises a probe binding to the third product, and
wherein a gene sequence of the third product to which the probe binds is a gene sequence complementary to an arbitrary gene sequence included in the second reverse primer.

8. The method of claim 7, wherein the probe is degraded according to gene synthesis by the third reverse primer to emit light.

9. A composition for a sequential polymerase chain reaction, comprising the template gene, DNA polymerase, dNTP, the first forward primer, the first reverse primer, the second reverse primer and the third reverse primer according to claim 1.

10. A kit for a sequential polymerase chain reaction, comprising the template gene, DNA polymerase, dNTP, the first forward primer, the first reverse primer, the second reverse primer and the third reverse primer according to claim 1.
